# DEMANDE DE BREVET EUROPEEN

(11) **EP 3 245 983 A1**
(43) Date de publication de la demande: **22.11.2017**
(21) Numéro de dépôt: 17170555.1
(22) Date de dépôt: 11.05.2017
(51) Int. Cl.: A61F 2/80

(54) **VALVE POUR EMBOÎTURE DE PROTHÈSE**

(30) Priorité: 18.05.2016 FR 1654391
(71) Demandeur: Chabloz Composants, 38170 Seyssinet-Pariset (FR)
(72) Inventeur: Chabloz, Pierre, 38450 Saint Georges de Commiers (FR)
(74) Mandataire: Hecké, Gérard

(57) **Abrégé**

Valve pour emboîture (2) de prothèse, l'emboîture (2) délimitant une cavité (6) ouverte pour recevoir un moignon (7) de membre amputé et délimitant un trou traversant (10) débouchant dans la cavité (6) et destiné à recevoir la valve, la valve comprenant un support (11) dans lequel un canal traversant (12) est formé et un organe (13) mobile entre une position d'évacuation dans laquelle l'organe (13) autorise un passage d'air hors du canal (12) selon une seule direction d'évacuation (A) et une position d'obturation dans laquelle l'organe (13) empêche un passage d'air à l'intérieur du canal (12) selon une direction (B) opposée à la direction d'évacuation (A), le support (11) comportant un moyen de fixation (14) configuré pour monter de manière amovible le support (11) au sein du trou traversant (10), et pour permettre un démontage du support (11) selon la direction (B) opposée à la direction d'évacuation (A).

## Description

### Domaine technique de l'invention

L'invention concerne les valves pour emboîture de prothèse, et plus particulièrement les prothèses pour membres inférieurs.

### État de la technique

Actuellement, on utilise des prothèses destinées à remplacer une partie manquante d'un membre amputé, tel qu'un bras ou une jambe. Les prothèses comportent une emboîture, c'est-à-dire une coque d'emboîtement, sur laquelle est fixée un membre prothétique. L'emboîture délimite une cavité ouverte pour recevoir le moignon du membre amputé. Par ailleurs, afin de maintenir l'emboîture solidaire avec le moignon, l'air résiduel situé entre l'emboîture et le moignon est chassé à travers un trou traversant ménagé dans l'emboîture. Plus particulièrement, une valve est logée dans le trou traversant pour empêcher une introduction d'air à l'intérieur de la cavité et pour autoriser une expulsion d'air à l'extérieur de la cavité. Une telle valve est du type anti-retour, ou unidirectionnelle, car elle permet l'écoulement d'air selon une seule direction en fonctionnement normal.

On peut citer par exemple, les demandes de brevet français FR2903294 et FR2903295 qui divulguent un bouchon pour prothèse, mobile entre une position d'obturation et une position de libération d'un trou traversant ménagé dans la prothèse. Le bouchon est équipé d'une valve d'évacuation d'air hors de la cavité de la prothèse et d'interdiction d'entrée d'air à l'intérieur de la cavité. La valve comporte un organe souple muni d'une ouverture qui est normalement fermée et qui s'ouvre sous l'effet d'une déformation de l'organe souple provoquée par une poussée d'air en provenance de la cavité de la prothèse.

On peut également citer la demande de brevet français FR2994381 qui divulgue un bouchon à valve automatique du type anti-retour pour une emboîture en forme de coque de prothèse à dépression, comportant une embase de fixation munie d'un embout tubulaire destiné à être assemblé à un orifice de l'emboîture, et munie d'un élément support de la valve d'évacuation d'air hors de l'emboîture et d'interdiction d'entrée d'air à l'intérieur de l'emboîture. L'élément support est mobile par pivotement pour occuper une position écartée où la valve est éloignée de l'orifice, et l'embase est équipée d'un bec de retenu de l'élément support. En outre, la valve est clipsée sur la partie supérieure de l'élément support.

Cependant ces bouchons à valve ne sont pas adaptés à tous les types de prothèses. En effet, les prothèses peuvent être recouvertes d'une esthétique en mousse et d'un bas de recouvrement en tissu, silicone, ou en polyuréthane. En outre, les valves qui viennent d'être décrites sont très sensibles à la poussière qui les empêche de fonctionner correctement. Il est donc nécessaire de nettoyer les valves régulièrement, et on doit alors démonter le bas de recouvrement et l'esthétique pour accéder aux valves.

On peut citer les documents de brevet US2015/0265433, FR1088509, WO2014/035561, US4595172, et US6613096 qui divulguent des valves pour emboîture de prothèse et pour lesquelles il est nécessaire de démonter l'esthétique de la prothèse afin de pouvoir nettoyer la valve.

### Objet de l'invention

L'objet de l'invention consiste à remédier à ces inconvénients, et plus particulièrement à fournir une valve facile à entretenir et qui garantit le maintien de la jonction entre l'emboîture et le moignon du membre amputé.

Un autre objet de l'invention consiste à fournir une emboîture de prothèse équipée d'une telle valve.

Selon un aspect de l'invention, il est proposé une valve pour emboîture de prothèse, l'emboîture délimitant une cavité ouverte pour recevoir un moignon de membre amputé et délimitant un trou traversant débouchant dans la cavité et destiné à recevoir la valve.

La valve comprend un support, dans lequel un canal traversant est formé, et un organe mobile entre une position d'évacuation dans laquelle l'organe autorise un passage d'air hors du canal selon une seule direction d'évacuation et une position d'obturation dans laquelle l'organe empêche un passage d'air à l'intérieur du canal selon une direction opposée à la direction d'évacuation.

Le support comporte un moyen de fixation configuré pour monter de manière amovible le support au sein du trou traversant.

Le moyen de fixation est configuré pour permettre un démontage du support selon la direction opposée à la direction d'évacuation.

On offre ainsi une valve dont l'organe à entretenir s'extrait par la cavité de l'emboîture. L'entretien de la valve est donc facilité car on évite de démonter l'esthétique qui enveloppe l'emboîture afin de pouvoir accéder à la valve.

La valve peut comprendre une embase configurée pour être montée au sein du trou traversant et comportant un corps creux taraudé, le moyen de fixation comprenant un filetage coopérant avec le taraudage du corps de l'embase.

Le support peut comporter une butée pour empêcher un démontage du support selon la direction d'évacuation.

La valve peut comprendre un actionneur configuré pour immobiliser l'organe dans la position d'évacuation afin d'autoriser un passage d'air hors du canal selon la direction d'évacuation et un passage d'air à l'intérieur du canal selon la direction opposée à la direction d'évacuation.

L'actionneur peut comporter une terminaison, et le support comporte une surface dans laquelle une ouverture du canal et une fente longitudinale située en regard de la terminaison sont formées, la fente longitudinale permettant de recevoir une partie déformable de l'organe pour éloigner l'organe de l'ouverture du canal.
La surface du support peut comporter deux zones planes situées de part et d'autre de la fente longitudinale, les deux zones planes étant inclinées l'une par rapport à l'autre.

La valve peut comporter un bouchon ayant une languette déformable sur laquelle est monté l'actionneur, la languette permettant un déplacement de l'actionneur pour que la terminaison presse l'organe.

Le support peut comporter un logement, la valve comprenant une pièce amovible configurée pour coopérer avec le logement afin de démonter le support selon la direction opposée à la direction d'évacuation.

Selon un autre aspect, il est proposé une emboîture de prothèse délimitant une cavité ouverte de réception d'un moignon de membre amputé et délimitant un trou traversant dans la cavité, l'emboîture comprenant une valve telle que définie ci-avant montée au sein du trou traversant.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
- la figure 1, illustre schématiquement une vue en coupe d'un mode de réalisation d'une valve montée sur une emboîture de prothèse selon l'invention ;
- la figure 2, illustre schématiquement une vue en coupe de la valve de la figure 1 dont le support est démonté ;
- la figure 3, illustre schématiquement une vue éclatée en coupe d'un autre mode de réalisation des différents éléments d'une valve ;
- la figure 4, illustre schématiquement des vues de profile en perspective de certains éléments de la figure 3 ;
- la figure 5, illustre schématiquement des vues de dessus en perspective des éléments de la figure 4 ;
- la figure 6, illustre schématiquement une vue de profile en perspective de la valve de la figure 3 dont les éléments sont montés ;
- la figure 7, illustre schématiquement une vue de dessus en perspective de la valve de la figure 6 ; et
- la figure 8, illustre schématiquement une autre vue de profile en perspective d'un actionneur de valve.

### Description détaillée

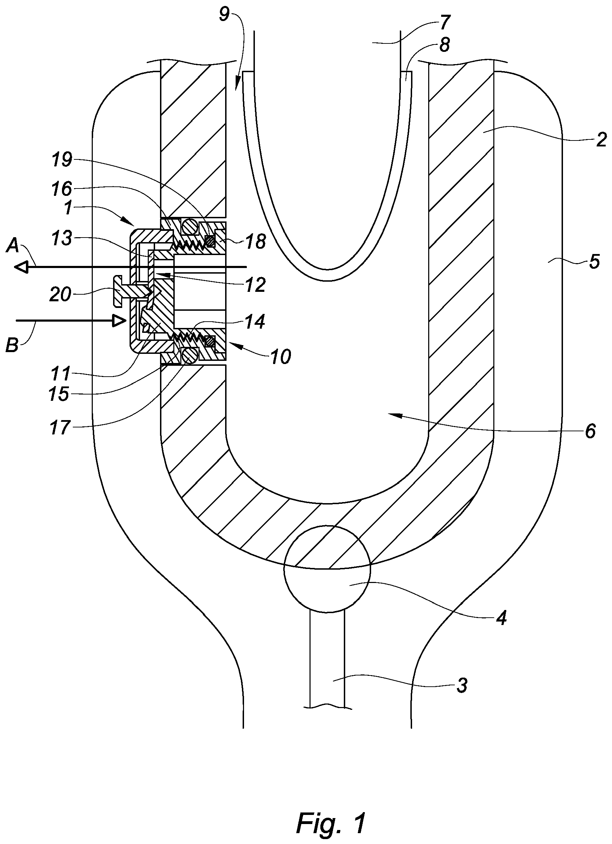

Sur la figure 1, on a représenté une valve 1 pour emboîture 2 de prothèse. La prothèse comporte un membre prothétique 3, par exemple une jambe ou un avant-bras de remplacement, fixé à une extrémité distale de l'emboîture 2 par une liaison articulée 4, comme une rotule. La prothèse peut également comprendre une esthétique 5 en mousse, en tissu, en silicone, ou en polyuréthane, recouvrant en partie, ou en totalité, l'emboîture 2. L'emboîture 2 délimite une cavité 6 ouverte pour recevoir un moignon 7 de membre amputé. Le moignon 7 du membre amputé peut être enveloppé d'un manchon 8 de forme extérieure complémentaire à la cavité 6 de l'emboîture 2, pour faciliter l'emboîtement du moignon 7 au sein de la cavité 6. Une ouverture 9 de la cavité 6 est située au niveau de l'extrémité proximale de l'emboîture 2. En outre, un trou traversant 10 est formé dans l'épaisseur de l'emboîture 2. Le trou traversant 10 débouche dans la cavité 6 et est conformé pour recevoir la valve 1.

La valve 1 est particulièrement adaptée pour une emboîture 2 de prothèse tibiale. La valve 1 permet de créer un vide entre l'emboîture 2 et le moignon 7, ce qui maintient leur jonction. Préférentiellement, la valve 1 est du type anti-retour, également notée valve unidirectionnelle, c'est-à-dire qu'en fonctionnement normal, la valve 1 laisse passer l'air selon une seule première direction A, en particulier depuis l'intérieur de la cavité 6 vers l'extérieur de la cavité 6, et empêche une entrée d'air à l'intérieur de la cavité 6 selon une deuxième direction B opposée à la première direction A. La première direction A est également appelée, « direction d'évacuation A », et la deuxième direction B est appelée « direction de démontage B ». De manière à maintenir l'emboîture 2 solidaire du moignon 7, l'air résiduel contenu dans la cavité 6 est chassé à travers la valve 1 à l'extérieur de la cavité 6, selon la première direction A. Lorsque la valve 1 est unidirectionnelle, elle empêche l'air extérieur de pénétrer dans la cavité 6, et un vide est alors créé entre le moignon 7 et l'emboîture 2 pour les maintenir solidairement l'un à l'autre. Avantageusement, pour garantir une étanchéité à l'air entre le moignon 7 et l'emboîture 2, un élément d'étanchéité peut être placé à l'extrémité proximale de l'emboîture 2. Par exemple, l'élément d'étanchéité peut être un joint à lèvre fixé sur l'emboîture 2, ou sur le manchon 8 recouvrant le moignon 7, ou être une gaine élastique enveloppant l'emboîture 2 et le manchon 7 au niveau de l'ouverture 9 de la cavité 6.

La valve 1 comporte un support 11, dans lequel un canal 12 est formé, et un organe 13 de fermeture du canal 12. Le canal 12 est traversant pour faire communiquer l'intérieur de la cavité avec l'extérieur. Lorsque la valve 1 est montée au sein du trou traversant 10, le canal traversant 12 débouche dans la cavité 6 pour permettre de chasser l'air résiduel situé entre l'emboîture 2 et le moignon 7. L'organe 13 de la valve 1 est mobile entre une position d'évacuation, dans laquelle l'organe 13 autorise un passage d'air hors du canal traversant 12 selon la direction d'évacuation A, et une position d'obturation, comme illustrée sur la figure 1, dans laquelle l'organe 13 empêche un passage d'air à l'intérieur du canal traversant 12 selon la direction de démontage B. L'organe 13 de la valve 1 est préférentiellement élastiquement déformable. Dans ce cas l'organe 13 se déforme afin d'occuper la position d'évacuation lorsque la pression de l'air contenu dans le canal traversant 12 est supérieure à la pression de l'air extérieur, et pour reprendre la position d'obturation lorsque la pression de l'air contenu dans le canal traversant 12 est inférieure à la pression de l'air extérieur. L'organe 13 peut être monté mobile sur le support 11. Préférentiellement, l'organe 13 comporte une première partie montée fixe sur le support 11 et une deuxième partie mobile par rapport au support 11. L'organe 13 peut en outre être élastiquement déformable. Par exemple, l'organe 13 peut être une membrane élastique.

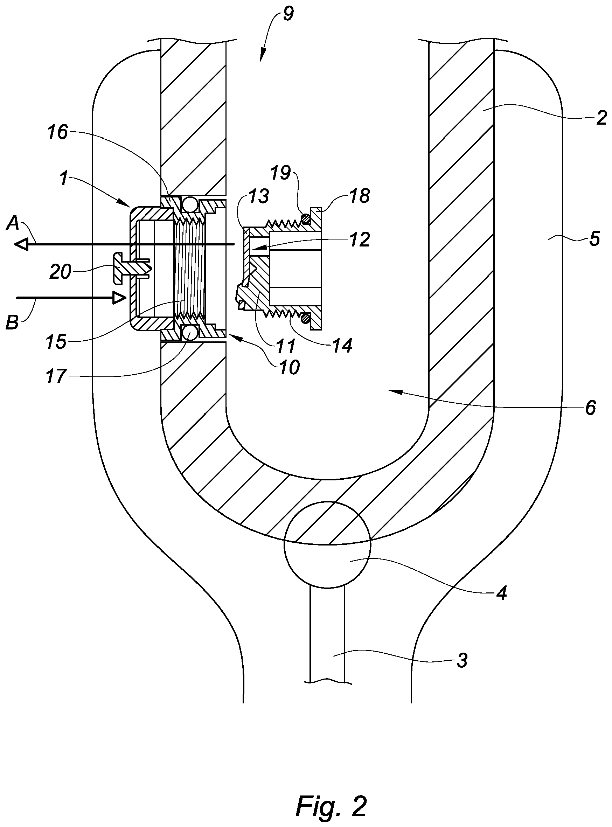

Le support 11 comporte également un moyen de fixation 14 configuré pour monter de manière amovible le support 11 au sein du trou traversant 10. Plus particulièrement, le moyen de fixation 14 est configuré pour permettre un démontage du support 11 selon la direction B opposée à la direction d'évacuation A. Ainsi on peut démonter l'organe 13 et son support 11 à l'intérieur de la cavité 6. On a illustré sur la figure 2, une valve 1 dont le support 11 et son organe 13 sont démontés selon la direction de démontage B et se situent à l'intérieur de la cavité 6. On évite alors d'avoir à démonter l'esthétique 5 pour retirer l'organe 13 de l'emboîture 2. Une telle valve 1 facilite son entretien régulier ou sa réparation. Par ailleurs, le moyen de fixation 14 permet un montage du support 11 selon la direction d'évacuation A. En d'autres termes, le support 11 est assemblé à l'emboîture 2 par l'intérieur de la cavité 6, c'est-à-dire en introduisant le support 11 et l'organe 13 à l'intérieur de la cavité 6. Ce qui facilite, en outre, son montage sur une prothèse munie au préalable d'une esthétique 5, car il n'est pas nécessaire de démonter l'esthétique 5 pour assembler le support 11 et l'organe 13 à l'emboîture 2.

Par exemple, le moyen de fixation 14 est configuré pour coopérer avec un moyen de réception 15 pour monter le support 11 dans le trou traversant 10. Le moyen de réception 15 peut être situé dans l'épaisseur de l'emboîture 2. On peut ainsi monter directement le support 11 sur l'emboîture 2. Avantageusement, la valve 1 peut comprendre une embase 16 configurée pour être montée au sein du trou traversant 10 et pour recevoir le support 11. L'embase 16 peut être vissée, soudée, collée, ou sertie dans l'épaisseur de l'emboîture 2. On peut prévoir un joint torique 17 logé dans une lumière prévue sur la surface extérieure de l'embase 16. Le joint torique 17 permet de garantir une étanchéité à l'air entre l'emboîture 2 et l'embase 16. L'embase 16 comporte un corps creux. En outre, l'embase 16 comporte le moyen de réception 15 pour monter le support 11 au sein du corps creux de l'embase 16. Lorsque l'embase 16 est fixée au trou traversant 10 elle empêche le support 11 de sortir inopinément quand le moignon 7 est retiré de la cavité 6. Par exemple, le moyen de fixation 14 comporte un filetage externe et le moyen de réception 15 comporte un taraudage interne. Le moyen de fixation 14 peut comprendre des crans de maintien et le moyen de réception 15 comprend des logements destinés à recevoir les crans de maintien.

Avantageusement, le support 11 peut comporter une butée 18 pour empêcher un démontage du support 11 selon la direction d'évacuation A. La butée 18 peut comprendre une collerette faisant saillie du corps du support 11. La collerette 18 est située à une extrémité proximale opposée à l'extrémité distale du support 11 où est situé l'organe 13. Le support 11 peut également comprendre une lumière prévue sur sa surface extérieure pour recevoir un joint torique supplémentaire 19. Le joint torique supplémentaire 19 permet de garantir une étanchéité à l'air entre le support 11 et l'embase 16.

La valve 1 peut encore comprendre un actionneur 20 configuré pour immobiliser l'organe 13 dans la position d'évacuation afin d'autoriser à la fois un passage d'air hors du canal traversant 12 selon la direction d'évacuation A, et un passage d'air à l'intérieur du canal traversant 12 selon la direction de démontage B. Lorsqu'on immobilise l'organe 13 dans la position d'évacuation, on permet une entrée d'air à l'intérieur de la cavité 6, ce qui désunit l'emboîture 2 et le manchon 7, pour que l'utilisateur puisse retirer la prothèse. On dit aussi que l'actionneur 20 déverrouille la valve 1. En effet, en fonctionnement normal, on dit que la valve 1 est verrouillée, et l'organe 13 est libre d'occuper la position d'obturation ou la position d'évacuation. L'organe 13 passe d'une position à l'autre en fonction de la différence de pression entre l'intérieur et l'extérieur de la cavité 6. Au contraire, on note que la valve 1 est déverrouillée lorsque l'organe 13 est maintenu dans la position d'évacuation et dans ce cas l'air peut circuler dans les deux directions A, B entre l'intérieur et l'extérieur de la cavité 6.

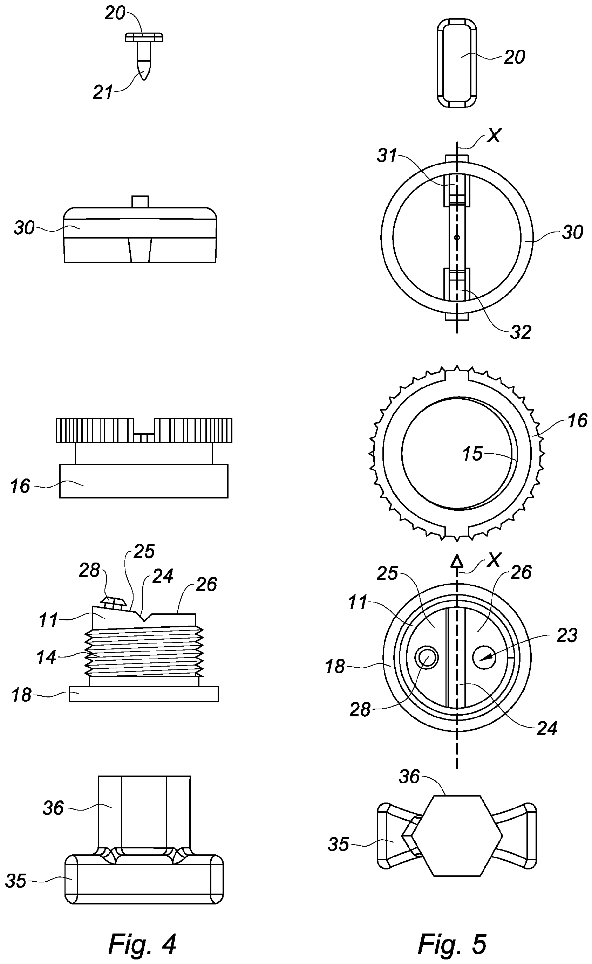

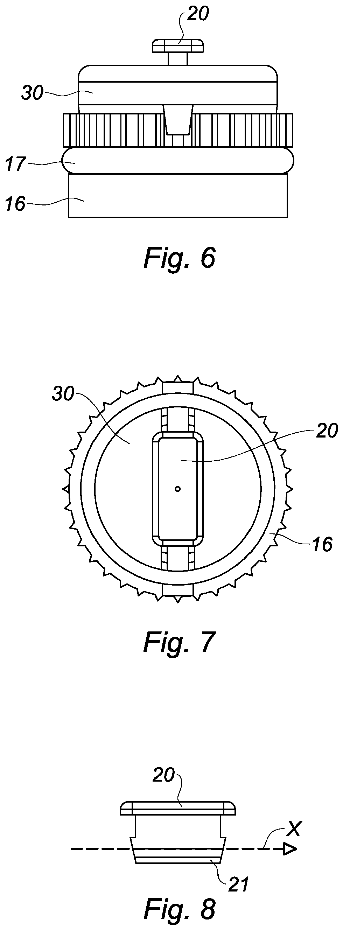

Sur la figure 2, on a représenté la valve 1, décrite à la figure 1, dont le support 11 est retiré de l'embase 16, c'est-à-dire retiré de l'emboîture 2. Sur les figures 4 et 5, on a représenté la valve 1 dont les éléments sont démontés, et sur les figures 6 et 7, on a représenté la valve 1 dont les éléments sont montés.

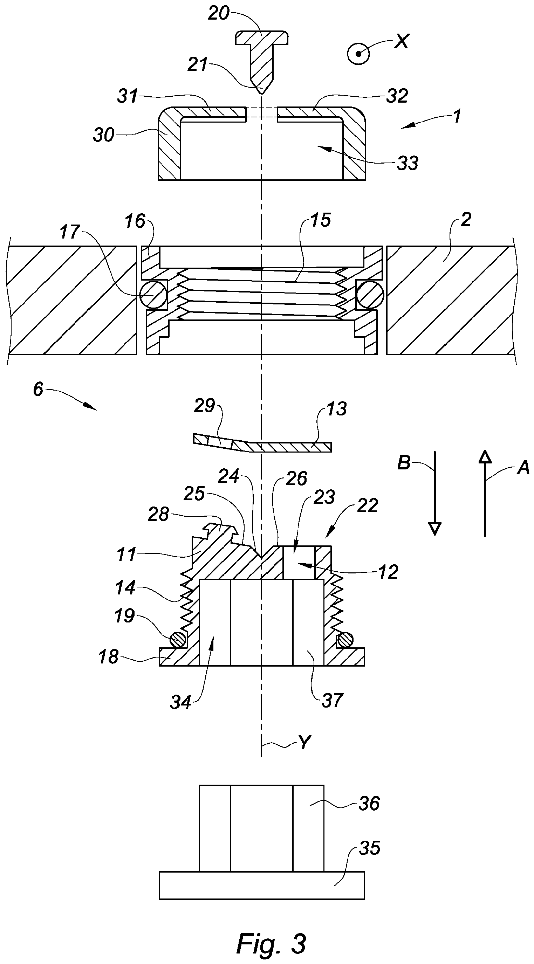

Sur la figure 3, on a représenté une vue éclatée des différents éléments de la valve 1. De préférence, l'actionneur 20 comporte une terminaison 21 et le support 11 comporte une surface 22 dans laquelle une ouverture 23 du canal traversant 12 et une fente 24 sont formées. La surface 22 est située à l'extrémité distale du support 11. La fente 24, a de préférence, une forme complémentaire à celle de la terminaison 21 de l'actionneur 20, pour recevoir une partie déformée de l'organe 13. Par exemple, la terminaison 21 de l'actionneur 20 s'étend selon un axe longitudinal X, perpendiculaire au plan de la feuille de la figure 3. Sur la figure 8, on a représenté une autre vue de l'actionneur 20 dans laquelle l'axe longitudinal X est dans le plan de la figure 8. La terminaison 21 peut avoir une forme en « V » pour fournir une extrémité en forme de pointe. La fente longitudinale 24 s'étend également selon un axe parallèle à l'axe longitudinal X de la terminaison 21. La fente 24 peut également avoir une forme en « V » complémentaire à celle de la terminaison 21. En particulier, la fente longitudinale 24 est située en regard de la terminaison 21. Lorsque l'actionneur 20 occupe une position initiale, comme illustrée à la figure 1, la valve 1 est verrouillée. Lorsque l'utilisateur presse l'actionneur 20, ce dernier translate selon un axe Y parallèle à un axe longitudinal du canal traversant 12. Lors de la translation, l'actionneur 20 presse l'organe 13 contre la surface 22 du support 11, en particulier au niveau de la fente longitudinale 24. Ainsi, une partie déformable de l'organe 13 est introduite au sein de la fente 24, ce qui soulève l'organe 13 et l'éloigne de l'ouverture 23 du canal traversant 12. En fin de translation, l'actionneur 20 occupe une deuxième position dans laquelle l'organe 13 occupe la position d'évacuation. L'organe 13 peut alors être maintenu dans la position d'évacuation en maintenant la pression de l'actionneur 20 contre l'organe 13. Lorsque l'organe 13 est immobilisé dans la position d'évacuation, l'air peut circuler depuis la cavité 6 vers l'extérieur, à travers le canal traversant 12, et dans le sens inverse, c'est-à-dire depuis l'extérieur vers l'intérieur de la cavité 6. Lorsque l'utilisateur relâche la pression sur l'actionneur 20, ce dernier reprend sa position initiale, et l'organe 13 reprend sa position d'obturation. L'organe 13 reprend sa position du fait de son élasticité.

Afin de faciliter la déformation de l'organe 13, pour qu'il occupe la position d'évacuation, la surface 22 du support 11 peut comporter deux zones planes 25, 26 situées de part et d'autre de la fente longitudinale 24. Les deux zones planes 25, 26 peuvent être inclinées l'une par rapport à l'autre. Par ailleurs, l'organe 13 peut avoir une forme incurvée qui coïncide avec les deux zones planes 25, 26 inclinées. Le support 11 peut comporter un ergot 28 qui s'insère dans un orifice 29 prévu dans l'organe 13. L'ergot 28 permet de maintenir fixe une partie de l'organe 13 pour maintenir l'organe 13 solidaire avec le support 11. L'autre partie de l'organe 13 étant déformable pour occuper les positions d'évacuation et d'obturation.

Avantageusement, la valve 1 comprend un bouchon 30 ayant au moins une languette 31, 32 déformable sur laquelle est monté l'actionneur 20. Les languettes déformables permettent le déplacement en translation de l'actionneur 20, selon l'axe Y, pour presser l'actionneur 20 contre l'organe 13. Par exemple, les languettes sont élastiques et l'actionneur 20 reprend sa position initiale, du fait de l'élasticité des languettes 31, 32. Selon un autre avantage, le bouchon 31 délimite une chambre 33. La chambre 33 est située à une extrémité de la valve 1, de sorte que l'organe 13 est situé entre la chambre 33 et le support 11. La chambre 33 permet à l'organe 13 d'occuper la position d'évacuation. En effet, la chambre 33 forme un volume de dégagement pour recevoir l'organe 13. Par ailleurs, un orifice traversant est formé dans la paroi du bouchon 30. L'orifice traversant est situé sur la surface du bouchon 30 où sont situées les languettes 31, 32. L'orifice traversant est débouchant dans la chambre 33 pour autoriser l'air extérieur de pénétrer dans la chambre 33, et inversement pour que l'air de la chambre 33 puisse passer vers l'extérieur.

Lorsque l'utilisateur souhaite retirer le moignon 7 de l'emboîture 2, après que le vide est créé entre l'emboîture 2 et le moignon 7, l'utilisateur presse l'actionneur 20, l'organe 13 est immobilisé dans la position d'évacuation, et l'air extérieur est introduit à l'intérieur de la cavité 6. Cet air introduit dans la cavité 6 désunit le moignon 7 et l'emboîture 2, et le moignon 7 peut être retiré. Par exemple, l'esthétique 5 peut être poreuse à l'air. Ainsi, lorsqu'on souhaite créer le vide entre l'emboîture 2 et le moignon 7, l'esthétique 5 permet de laisser s'échapper l'air résiduel situé entre l'emboîture 2 et le moignon 7 vers l'extérieur. Par ailleurs, lorsqu'on souhaite retirer le moignon 7 de l'emboîture 2, l'esthétique 5 permet à l'air extérieur de s'introduire dans la cavité 6. En variante, on peut prévoir un canal d'évacuation d'air situé dans l'épaisseur de l'esthétique 5 et reliant la chambre 33 avec l'extérieur. En particulier, le canal d'évacuation relie l'orifice traversant du bouchon 30 avec l'extérieur. Avantageusement, l'esthétique 5 est souple pour permettre à l'utilisateur de presser l'actionneur 20, depuis l'extérieur, en pressant l'esthétique 5.

Le support 11 peut également comporter un logement 34 destiné à recevoir une pièce amovible 35. La pièce 35 est introduite dans le logement 34, et coopère avec le logement 34 pour démonter le support 11 selon la direction de démontage B. Par exemple, la pièce 35 peut comporter plusieurs pans 36 et le logement 34 comporte des pans complémentaires pour pouvoir mettre en rotation le support 11, autour de l'axe Y, et ainsi démonter le support 11 de la valve 1.

## Revendications

1. Valve pour emboîture (2) de prothèse, l'emboîture (2) délimitant une cavité (6) ouverte pour recevoir un moignon (7) de membre amputé et délimitant un trou traversant (10) débouchant dans la cavité (6) et destiné à recevoir la valve, la valve comprenant un support (11) dans lequel un canal traversant (12) est formé et un organe (13) monté mobile entre une position d'évacuation dans laquelle l'organe (13) autorise un passage d'air hors du canal (12) selon une seule direction d'évacuation (A) et une position d'obturation dans laquelle l'organe (13) empêche un passage d'air à l'intérieur du canal (12) selon une direction (B) opposée à la direction d'évacuation (A), le support (11) comportant un moyen de fixation (14) configuré pour monter de manière amovible le support (11) au sein du trou traversant (10), le moyen de fixation (14) étant configuré pour permettre un démontage du support (11) selon la direction (B) opposée à la direction d'évacuation (A), **caractérisée en ce qu'**elle comprend une embase (16) configurée pour être montée au sein du trou traversant (10) et comportant un corps creux taraudé (15), le moyen de fixation (14) comprenant un filetage coopérant avec le taraudage (15) du corps de l'embase (16).

2. Valve selon la revendication 1, dans laquelle le support (11) comporte une butée (18) pour empêcher un démontage du support (11) selon la direction d'évacuation (A).

3. Valve selon la revendication 1 ou 2, comprenant un actionneur (20) configuré pour immobiliser l'organe (13) dans la position d'évacuation afin d'autoriser un passage d'air hors du canal (12) selon la direction d'évacuation (A) et un passage d'air à l'intérieur du canal (12) selon la direction (B) opposée à la direction d'évacuation (A).

4. Valve selon la revendication 3, dans laquelle l'actionneur (20) comporte une terminaison (21), et le support (11) comporte une surface (22) dans laquelle une ouverture (23) du canal (12) et une fente longitudinale (24) située en regard de la terminaison (21) sont formées, la fente longitudinale (24) permettant de recevoir une partie déformable de l'organe (13) pour éloigner l'organe (13) de l'ouverture (23) du canal (12).

5. Valve selon la revendication 4, dans laquelle la surface (22) du support (11) comporte deux zones planes (25, 26) situées de part et d'autre de la fente longitudinale (24), les deux zones planes (25, 26) étant inclinées l'une par rapport à l'autre.

6. Valve selon la revendication 4 ou 5, comprenant un bouchon (30) ayant une languette déformable (31, 32) sur laquelle est monté l'actionneur (20), la languette (31, 32) permettant un déplacement de l'actionneur (20) pour que la terminaison (21) presse l'organe (13).

7. Valve selon l'une des revendications 1 à 6, dans laquelle le support (11) comporte un logement (34), la valve comprenant une pièce (35) amovible configurée pour coopérer avec le logement (34) afin de démonter le support (11) selon la direction (B) opposée à la direction d'évacuation (A).

8. Emboîture (2) de prothèse délimitant une cavité (6) ouverte de réception d'un moignon (7) de membre amputé et délimitant un trou traversant (10) dans la cavité (6), l'emboîture (2) comprenant une valve selon l'une des revendications 1 à 7 montée au sein du trou traversant (10).
